# EUROPEAN PATENT APPLICATION

(11) **EP 4 328 859 A1**
(43) Date of publication of application: **28.02.2024**
(21) Application number: 23185254.2
(22) Date of filing: 13.07.2023
(51) Int. Cl.: G06T 11/00

(54) **METHOD AND SYSTEM FOR REMOVING FOREIGN MATERIAL FROM IMAGES**

(30) Priority: 26.08.2022 AU 2022221522
(71) Applicant: CurveBeam Al Limited, Melbourne VIC 3000 (AU)
(72) Inventor: Peng, Yu, Melbourne, 3000 (AU); Xiaoxu, Li, Melbourne, 3000 (AU)
(74) Representative: Fleuchaus & Gallo Partnerschaft mbB

(57) **Abstract**

A method and system for training a machine learning model for reducing or removing a foreign material or artefacts due to a foreign material from an image of a subject, the method comprising: generating one or more first simulated images from one or more real or simulated images of the foreign material (and optionally artefacts due to the foreign material), and from one or more real images of one or more subjects that are free of the foreign material and of artefacts due to the foreign material, such that the generated simulated images include the foreign material and artefacts due to the foreign material; generating one or more predicted images employing at least the first simulated images with a machine learning network that implements a machine learning model; and training or updating the machine learning model with the machine learning network by reducing or minimizing a difference between the one or more predicted images and ground truth data comprising one or more real or simulated images.

## Description

### Related Application

The present application claims the benefit of the filing date of Australian Patent Application No. 2022221522.

### Field of the invention

The present invention relates to a method and system for reducing or removing foreign material and foreign material artefacts (such as metal and metal artefacts) from scans or images, such as medical images and in particular from Computed Tomography (CT) imaging, Magnetic Resonance (MR) imaging and x-ray imaging.

### Background

Medical imaging modalities, such as x-ray, computed tomography (CT) and MRI, greatly facilitate medical diagnosis. They provide clear views of human organs and tissues to assist the medical analysis and clinical diagnosis, but a significant problem is the presence of foreign material-especially in the form of metal and metal artefacts (caused by the presence of the metal) apparent in scans of subjects or patients.

Metal artefacts arise from the presence of metal, typically-though not exclusively-in the form of metal implants or metal protheses of, for example, titanium alloy, cobalt-chromium alloy and stainless steel (being especially suitable for orthopedic applications owing to their high tensile strength, strength under compression and stiffness), as well as dental amalgam and silver. More recently, implants and protheses of biomaterials have also been developed, including those of ceramics, glasses, polymers, composites, glass-ceramics and metal alloys, and artefacts can also arise from those materials.

Artefacts are commonly generated by beam hardening, scattering, photon starvation, and patient motion. Metal and metal artefacts typically appear as ultra-bright, dark or streak-like areas, polluting or obscuring features of interest in a scan. This can significantly compromise the diagnosis or the monitoring of an injury, pathology or treatment. Hence, metal and metal artefact reduction or removal aims to reduce or remove metal and metal artefacts from scans and consequently, to a degree, to recover the otherwise 'polluted' or obscured areas. Metal artefact reduction/removal in particular, but also metal reduction/removal, can thus be important parts of a medical image analysis system.

Existing methods of metal artefact reduction/removal fall into four categories: (i) physical effects correction-based methods [1][2], (ii) interpolation-based methods [3][4], (iii) iterative reconstruction-based methods [5][6], and (iv) deep learning-based methods [7][8]. The first entails correcting the physical effects but fails with large metal parts. Interpolation-based methods replace metal projections using interpolation algorithms, but the interpolation usually introduces new artefacts. Iterative reconstruction-based methods iteratively remove the metal artefacts and reconstruct the unaffected projections via proper regularizations, but unsatisfactory results usually appear owing to variations in metal sizes, positions and materials. Deep learning method learn the artefact removal strategy via massive data, but the metal-corrupted regions are still not well recovered by existing deep learning methods.

A major problem is that algorithms developed on one body part cannot be applied to another, owing to the different characteristics of human body parts. In addition, secondary artefacts-caused by, for example, interpolation and some deep learning algorithms-are often introduced with large metal inserts in CT. Furthermore, medical CT images of the same subjects with and without metal artefacts are hard to obtain for use in training machine learning models, and this lack of training data limits the performance of machine learning-based methods.

### Summary

It is an object of the present invention to provide a method and system for training a machine learning model for reducing or removing a foreign material or artefacts due to a foreign material from an image of a subject, such as a medical image (e.g. an x-ray, CT or MRI image that includes bone) and such as a foreign material in the form of a metal or metals.

According to a first aspect of the invention, there is provided a method for training a machine learning model for reducing or removing at least one foreign material or artefacts due to the foreign material from an image, the method comprising:
generating one or more first simulated images from one or more real or simulated images of the foreign material, and from one or more real images of one or more subjects that are free of the foreign material and of artefacts due to the foreign material, such that the generated simulated images include the foreign material and artefacts due to the foreign material;
generating one or more predicted images employing at least the first simulated images with a machine learning network that implements a machine learning model; and
training or updating the machine learning model with the machine learning network by reducing or minimizing a difference between the one or more predicted images and ground truth data comprising one or more real or simulated images.

It should be understood that the terms "image" and "scan" as used herein are essentially synonymous. In practice, a scan may comprise more than one image; however, the present invention is adapted to be employed to process one or more images, so even that distinction is generally moot. It should also be understood that a reference to an image or scan that contains or includes a foreign material refers to that image or scan including a full or partial image of that foreign material (or of an object made that foreign material). Furthermore, the foreign material need not be of a single substance. For example, the foreign material may be an alloy (as mentioned herein) so comprise a plurality of metals, or the foreign material may be the form of a plurality of objects (or portions thereof) of the same material or of different materials.

Additionally, foreign material is not limited to metallic materials. As used herein, it may comprise any dense material that causes beam hardening, scattering, photon starvation, noise etc., thus producing discernible artefacts such as streaks, dark holes, blurriness etc., on the images and so degrade image quality.

The expression "foreign material and foreign material artefact-free" as used herein means free of both the foreign material(s) and of artefacts arising from that or those foreign materials, where the "foreign material(s)" is or are those to be removed or that have been removed from the image(s)/scan(s). Similarly, the expression "foreign material and foreign material artefact removed/removal" as used herein refers to image(s)/scan(s) from which foreign material and foreign material artefacts have been removed, or a feature (such as a deep learning model or network) configured for the removal of-or for training the removal of-foreign material(s) and/or foreign material artefacts from image(s)/scan(s). Furthermore, it should be understood that "free" (such as in "foreign material free" or "artefact free") means omitted or excluded to the extent possible.

As stated above, the first simulated images may be generated from one or more real or simulated images of the foreign material and artefacts due to the foreign material. If simulated images are employed, they may be generated, for example, by segmenting one or more regions of the foreign material(s) (e.g. metal) from real scans-such as CT scans-that contain the foreign material(s). In another example, a metal mask image or images can be generated for use in this manner by simulating clusters of bright pixels/voxels placed on a black or blank image.

Optionally, in each embodiment, the one or more real or simulated images of the foreign material also include artefacts due to the foreign material. For example, if these images are real images, they are likely to include both foreign material(s) and artefacts due to the foreign material(s). However, the method may also include generating these images by segmenting foreign material regions from real images with both foreign material and artefacts, such that the resulting images have foreign material only.

If the one or more real or simulated images of the foreign material are simulated images, they can include foreign material(s) or foreign material(s) and artefacts thereof.

In an embodiment, the one or more predicted images are free of both the foreign material and artefacts due to the foreign material, and the ground truth data comprises one or more real images of one or more subjects that are free of the foreign material and of artefacts due to the foreign material, and the machine learning model is configured to reduce or remove a foreign material and artefacts due to a foreign material from an image.

The method may further comprise optimizing the trained machine learning model with a discriminator network configured to discriminate between the one or more predicted images and one or more real images free of both the foreign material(s) and of artefacts due to the foreign material(s).

Thus, the less differentiation the discriminator network can find between the predicted MAR image(s) and the real or simulated images, the better is the machine learning model optimized. Once optimized, the machine learning model reduces the foreign material and/or artefacts thereof in the image(s)/scan(s), including secondary artefacts introduced by the prediction process in the predicted images.

In another embodiment, the method further comprises generating one or more second simulated images from the one or more real or simulated images of the foreign material, and from the one or more real images of the one or more subjects that are free of the foreign material and of artefacts due to the foreign material, such that the generated second simulated images include the foreign material;
wherein the one or more predicted images are free of artefacts due to the foreign material, the ground truth data comprises the second simulated images, and the machine learning model is configured to reduce or remove artefacts due to the foreign material from an image.

In another embodiment, the method further comprises generating one or more second simulated images from the one or more real or simulated images of the foreign material, and from the one or more real images of the one or more subjects that are free of the foreign material and of artefacts due to the foreign material, such that the generated second simulated images include artefacts due to the foreign material;
wherein the one or more predicted images are free of the foreign material, the ground truth data comprises the second simulated images, and the machine learning model is configured to reduce or remove the foreign material from an image.

The foreign material may be, for example, titanium alloy, cobalt-chromium alloy, steel, stainless steel, dental amalgam, silver or other metal, a ceramic, glass, polymeric, a composite, a glass-ceramic, or a biomaterial.

The machine learning model may be configured to reduce or remove a plurality of foreign materials and/or artefacts due to the foreign materials from an image

The method may include annotating or labelling the one or more first simulated images.

According to a second aspect of the invention, there is provided a system for training a machine learning model for reducing or removing a foreign material or artefacts due to a foreign material from an image, the system comprising:
an image simulator configured to generate one or more first simulated images from one or more real or simulated images of the foreign material, and from one or more real images of one or more subjects that are free of the foreign material and of artefacts due to the foreign material, such that the generated simulated images include the foreign material and artefacts due to the foreign material;
a machine learning network configured to generate one or more predicted images employing at least the first simulated images, the machine learning network implementing a machine learning model;
wherein the machine learning network is configured to reduce or minimize a difference between the one or more predicted images and ground truth data comprising one or more real or simulated images.

Optionally, the one or more real or simulated images of the foreign material also include artefacts due to the foreign material.

In an embodiment, the one or more predicted images are free of both the foreign material and artefacts due to the foreign material, and the ground truth data comprises one or more real images of one or more subjects that are free of the foreign material and of artefacts due to the foreign material, such that the machine learning model is configured to reduce or remove a foreign material and artefacts due to a foreign material from an image.

The system may be configured to optimize the trained machine learning model with a discriminator network configured to discriminate between the one or more predicted images and one or more real images free of both the foreign material(s) and of artefacts due to the foreign material(s).

In an embodiment, the image simulator is further configured to generate one or more second simulated images from the one or more real or simulated images of the foreign material, and from the one or more real images of the one or more subjects that are free of the foreign material and of artefacts due to the foreign material, such that the generated second simulated images include the foreign material;
wherein the one or more predicted images are free of artefacts due to the foreign material, and the ground truth data comprises the second simulated images, such that the machine learning model is configured to reduce or remove artefacts due to the foreign material from an image.

In another embodiment, the image simulator is further configured to generate one or more second simulated images from the one or more real or simulated images of the foreign material, and from the one or more real images of the one or more subjects that are free of the foreign material and of artefacts due to the foreign material, such that the generated second simulated images include artefacts due to the foreign material;
wherein the one or more predicted images are free of the foreign material, and the ground truth data comprises the second simulated images, such that the machine learning model is configured to reduce or remove the foreign material from an image.

The foreign material may be, for example, titanium alloy, cobalt-chromium alloy, steel, stainless steel, dental amalgam, silver or other metal, a ceramic, glass, polymeric, a composite, a glass-ceramic, or a biomaterial.

The system may be configured to train the machine learning model to reduce or remove a plurality of foreign materials and/or artefacts due to the foreign materials from an image.

The system may further comprise an annotator configured or operable to receive annotations or labels for features of the one or more first simulated images.

According to a third aspect of the invention, there is provided a method for reducing or removing at least one foreign material or artefacts due to the foreign material from an image, the method comprising: reducing or removing from an image of a subject at least one foreign material or artefact due to the foreign material, or both the at least one foreign material and the artefact due to the foreign material, using a machine learning model trained according to the method of the first aspect.

According to a fourth aspect of the invention, there is provided a system for reducing or removing at least one foreign material or artefacts due to the foreign material from an image, the system being configured to reduce or remove from an image of a subject at least one foreign material or artefact due to the foreign material, or both the at least one foreign material and the artefact due to the foreign material, using a machine learning model trained according to the method of the first aspect.

According to a fifth aspect of the invention, there is provided a computer program comprising program code configured, when executed by one of more computing devices, to implement the method of the first and/or third aspects (and any of their embodiments). According to this aspect, there is also provided a computer-readable medium (which may be non-transient), comprising such a computer program.

It should be noted that any of the various individual features of each of the above aspects of the invention, and any of the various individual features of the embodiments described herein, including in the claims, can be combined as suitable and desired.

### Drawings

In order that the invention may be more clearly ascertained, embodiments will now be described by way of example with reference to the following drawing, in which:
Figure 1 is a schematic view of an image processing system in the form of a medical image processing system according to an embodiment of the present invention.
Figure 2 depicts the general workflow of the system of Figure 1.
Figure 3A depicts a deep learning architecture configured to produce models for foreign material and foreign material artefact removal according to an embodiment of the present invention.
Figure 3B depicts a deep learning architecture configured to produce models for foreign material artefact removal according to an embodiment of the present invention.
Figure 3C depicts a deep learning architecture configured to produce models for foreign material removal according to an embodiment of the present invention.
Figure 4 is a schematic flow diagram of the training of the deep learning model(s) for foreign material and foreign material artefact removal according to an embodiment of the present invention.
Figure 5 is a schematic flow diagram of an exemplary generation of a simulated metal and metal artefact scan.
Figures 6A to 6D are examples of real metal-free CT and corresponding simulated metal and metal artefact CT scans.
Figures 7A to 7D are inverted versions of the images of Figures 6A to 6D, respectively.
Figures 8A to 8D are details of the images of Figures 6A to 6D, respectively.
Figure 9 depicts an embodiment of the metal and metal artefact removal network of the deep learning architecture of Figure 3A.
Figure 10 depicts an embodiment of the discriminator network of the deep learning architecture of Figure 3A.
Figure 11 is a schematic flow diagram of the training of a deep learning model for foreign material artefact removal according to an embodiment of the present invention.
Figures 12A, 12B and 12C are, respectively, a CT image of a foot in raw form, the same image with metal artefacts removed, and the same image with both metal and metal artefacts removed.
Figures 13A, 13B and 13C are inverted versions of the images of Figures 12A, 12B and 12C, respectively.

### Detailed description

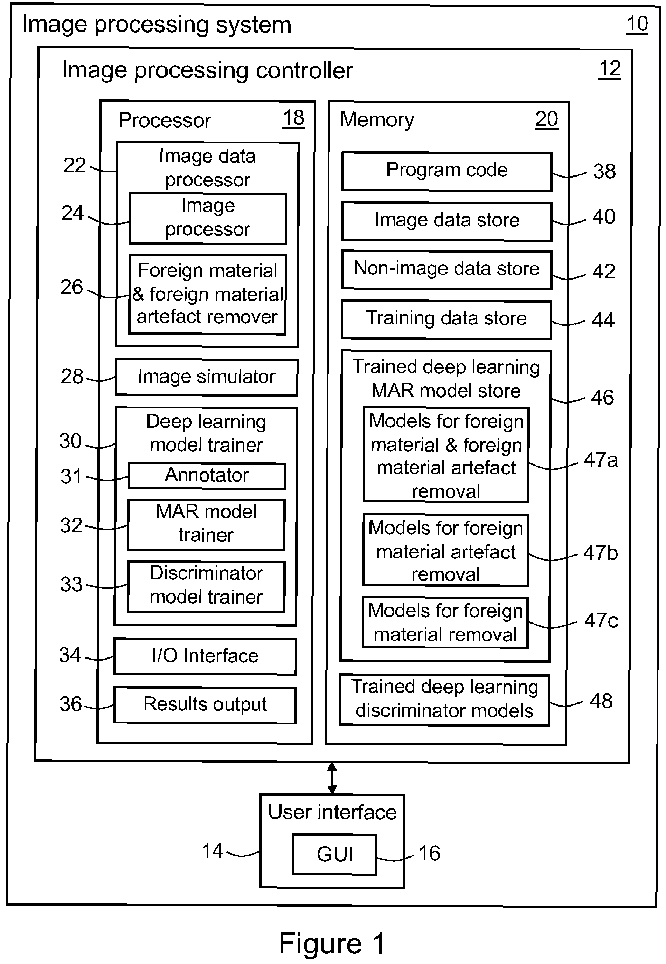

Figure 1 is a schematic view of an image processing system in the form of a medical image processing system 10 according to an embodiment of the present invention. System 10 is configured to remove one or more foreign materials and/or foreign material artefacts from an image or scan. The foreign materials, in this embodiment, are typically metals such as steel, titanium alloy or cobalt-chromium alloy, but system 10 is also suitable for use with other materials such as dental amalgam, silver, ceramics, glass, polymers, composites, glass-ceramics, and biomaterials. In each case, system 10 is trained using training images that include or simulate the respective material(s) and/or artefacts.

Referring to Figure 1, system 10 comprises an image processing controller 12 and a user interface 14 (including a GUI 16). The user interface 14 typically consists of one or more displays (on one or more of which may display the GUI 16), a keyboard and a mouse, and optionally a printer. Image processing controller 12 includes at least one processor 18 and memory 20. System 10 may be implemented, for example, as a combination of software and hardware on a computer (such as a server, personal computer or mobile computing device) or as a dedicated image processing system. System 10 may optionally be distributed; for example, some or all of the memory components may be located remotely from the processor; user interface may be located remotely from memory and/or from processor and, indeed, may comprise a web browser or a mobile device application.

Memory 20 is in data communication with processor 18 and typically comprises both volatile and non-volatile memory (and may include more than one type of memory), including RAM (Random Access Memory), ROM and one or more mass storage devices.

As discussed in greater detail below, processor 18 includes an image data processor 22, which includes an image processor 24 and a foreign material and foreign material artefact remover 26 (also referred to herein as Material and Artefact remover 26 or MA remover 26) configurable to remove one or more foreign materials and/or foreign material artefacts from an image or scan, employing various other components of controller 12 as detailed below. That is, according to the nature of the training data, MA remover 26 removes from an image or scan (a) one or more foreign materials, or (b) foreign material artefacts, or (c) both foreign materials and foreign material artefacts.

Processor 18 also includes an image simulator 28 configured to generate simulated images, such as by simulating a foreign material and/or with foreign material artefacts in an existing image that lacks one or both of those.

Processor 18 further includes a deep learning model trainer 30 (which includes an annotator 31, a MAR model trainer 32 and a discriminator model trainer 33), an I/O interface 34 and an output in the form of a results output 36. Deep learning model trainer 30 is configured to train MAR models and discriminator models, and can perform functions such as applying mean square loss calculations to scans and determining the performance of a discriminator network by determining and outputting cross-entropy loss.

Annotator 31 is user-operable to annotate or label features (or to input annotations or labels thereof) that are discernable in images/scans.

Memory 20 includes program code 38, an image data store 40, a non-image data store 42, a training data store 44, a trained deep learning MAR model store 46 of MAR models, and one or more trained deep learning discriminator models 48.

The MAR models may be configured to minimize or remove foreign material and/or foreign material artefacts from images/scans (according to the training data used to train the respective model). Thus, MAR model store 46 may include one or more models 47a trained to remove foreign material(s) and foreign material artefacts, one or more models 47b trained to remove foreign material artefacts, and/or one or more models 47c trained to remove foreign material(s).

In use, system 10 feeds an image or scan (such as a CT scan) that includes one or more foreign materials (such as a metal) and one or more artefacts thereof into one or more of the trained MAR model(s) 47a, 47b, 47c. MAR model(s) 47a, 47b, 47c are adapted to process the scan and output a scan with the foreign material and/or foreign material artefact(s) reduced or removed. System 10 may also use the discriminator model(s) 48 for model optimization in further or subsequent training of models 47a for foreign material(s) and foreign material artefacts removal.

Image processing controller 12 is implemented, as least in part, by the processor 18 executing program code 38 from memory 20.

In broad terms, the I/O interface 34 is configured to read or receive image data and non-image data (such as in DICOM format) pertaining to subjects or patients or imaging modalities into image data store 40 and non-image data store 42 of memory 20 for processing. Image processor 24 of image data processor 22 is configured to pre-process the images, such as by image format conversion and pixel/voxel values standardization, and/or to post-process the images, such as by image noise removal and/or image visualization enhancement. MA remover 26 of image data processor 22 is configured to remove the foreign material artefacts and/or foreign material from the images. I/O interface 34 outputs the results of this processing to, for example, results output 36 and/or to GUI 16.

Referring to Figure 1, system 10 is configured to receive two types of data: image data and non-image data. Image data are scans of subjects or patients, which scans include foreign material and foreign material artefacts. Non-image data include information about the imaging modalities of the scans, such as the scanning parameters of the imaging modality of each scan and information indicative of the desired processing/results (such as the reduction or removal of both foreign material and foreign material artefacts, the reduction or removal of foreign material only, or the reduction or removal of foreign material artefacts only).

These scanning parameters are suitable for use in the simulation of the metal artefacts. For example, CT scanning parameters (such as the CT image resolution, sensor spacing, x-ray rotation angle increment, and distance from the x-ray source to the centre of rotation) are needed for sinogram conversion in the simulation of MA CT scans.

System 10 stores the image data and non-image data in image data store 40 and non-image data store 42, respectively.

As has been seen, image data processor 22 comprises two components: image processor 24 and MA remover 26. The image data and non-image data are accessed by image data processor 22. Based on the imaging information (e.g. scanning modality and parameters) and desired processing/results from the non-image data, MA remover 26 selects one or more models 47a, 47b, 47c from trained deep learning model store 46 for use in reducing or removing foreign material and/or foreign material artefacts from the scans. Image processor 24 may use image processing algorithms to pre-process and/or post-process the scan images.

In one embodiment, one model of removing foreign material and foreign material artefacts is trained. The trained MAR model 47a removes both the foreign material and foreign material artefacts from scans. In another embodiment two models are trained, a first model 49b for removing foreign material artefacts and a second model 47c for removing foreign material, respectively.

In one embodiment, a different foreign material and foreign material artefact removal model is trained for each imaging modality, such as for each of x-ray, CT and/or MRI. In another embodiment, more than one foreign material and foreign material artefact removal model is trained for each type of imaging modality. In still another embodiment, one or more foreign material and foreign material artefact removal models are trained for each type of imaging modality. For example, a separate model may be trained to suit respective different scanning settings of one CT scanner.

The training data are prepared for training the foreign material and foreign material artefact removal models and discriminator models. The training data comprise real foreign material and artefact-free scans, real foreign material and artefact scans and simulated foreign material and artefact scans.

Processor 18 includes a deep learning model trainer 30 configured to train foreign material removal models, foreign material artefact removal models, foreign material and foreign material artefact removal (MAR) models and discriminator models (and retrain or update such trained deep learning models, as discussed below) using the training data. In other embodiments, however, a deep learning model trainer may be configured or used only to retrain or update trained deep learning models.

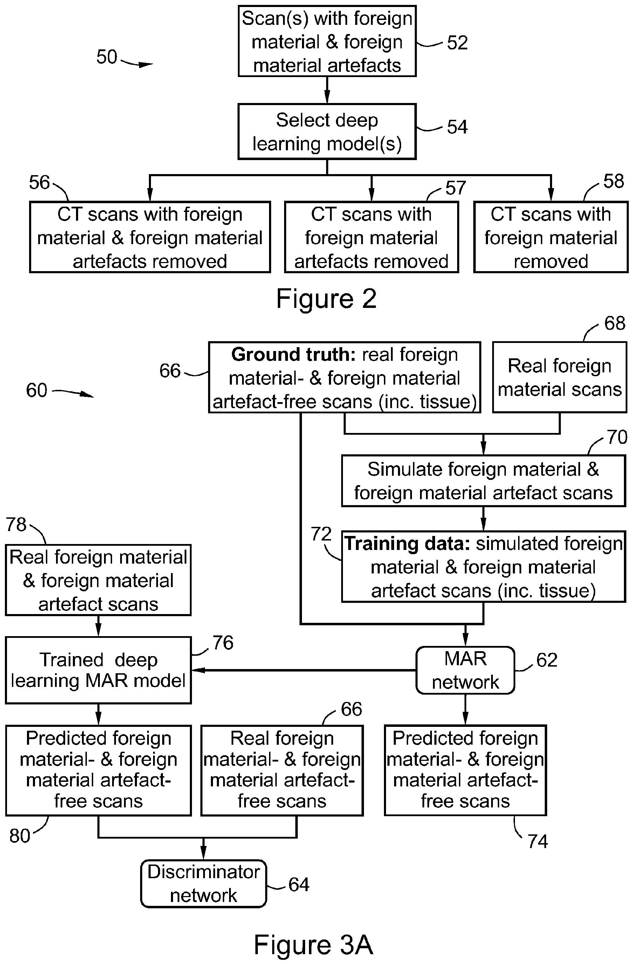

Figure 2 depicts the general workflow 50 of system 10 of Figure 1. Referring to Figure 2, at step 52, scans with foreign material and foreign material artefacts are read into memory 20 of system 10. Memory 20 is preferably configured to allow high-speed access of data by system 10. For example, if system 10 is implemented as a combination of software and hardware on a computer, the images may be written into and read from RAM of memory 20.

At step 54, image data processor 22 selects one or more suitable deep learning models 47a, 47b, 47c from trained deep learning model store 46 in memory 20. The deep learning model selection is based on the imaging modality information and the desired information as stipulated in the non-image data (i.e. stipulating whether foreign material and/or artefacts should be removed from the images/scans). For example, if the scan or scans are CT images, image data processor 22 selects deep learning model(s) trained using CT scan data; to remove both foreign material and foreign material artefacts, a model 47a trained for both foreign material removal and foreign material artefact removal is selected; to remove foreign material artefacts only, a model 47b trained for metal artefact removal only is selected; to remove foreign material only, a model 47c trained for foreign material removal only is selected.

At step 56, MA remover 26 generates-on the basis of the selected trained deep learning models 47a and input CT images-CT images with the foreign material and foreign material artefacts removed. Additionally or alternatively, at step 57, MA remover 26 generates-on the basis of the selected trained deep learning models 47b and input CT images-CT images with only the foreign material artefacts removed. Additionally or alternatively, at step 58, MA remover 26 generates-on the basis of the selected trained deep learning models 47c and input CT images-CT images with only the foreign material removed.

Figure 3A depicts the deep learning architecture 60 (implemented by deep learning model trainer 30) that trains models 47a for foreign material and foreign material artefact removal. Referring to Figure 3A, the deep learning architecture 60 includes two networks: foreign material and/or foreign material artefact removal (MAR) network 62 and discriminator network 64. Deep learning MAR models 47a are trained by deep learning model trainer 30 from MAR network 62 through supervised learning, and models 47a are optimized with discriminator network 64 (using discriminator models 48) through unsupervised learning.

MAR network 62 outputs trained models 47a in a method that involves comparing and minimizing the difference between predicted scans made by MAR network 62 and the ground truth (in this example, real foreign material- & foreign material artefact-free scans 66), as follows.

Firstly, image simulator 28 uses real foreign material (e.g. metal) and foreign material artefact-free scans 66 (i.e. free of both the foreign material(s) and artefacts due to the foreign material(s), created using a particular imaging modality) and real foreign material scans 68 (created using the same imaging modality) to generate 70 simulated scans 72 with foreign material and foreign material artefacts. The real foreign material and foreign material artefact-free scans 66 may be regarded-in this example-as the ground truth, while the simulated foreign material and foreign material artefact scans 72 may be regarded as the training data (and stored in training data store 44 is desired).

The simulated scans 72 are annotated (using annotator 31), then inputted into MAR network 62. The output of MAR network 62 comprises predicted foreign material and foreign material artefact-free (MAR) scans 74. MAR network 62 trains a deep learning MAR model 76 by minimizing the difference between the predicted MAR scans 74 and the real foreign material and foreign material artefact-free scans 66.

The MAR model 76, once trained by MAR network 62, is optimized by discriminator network 64 as follows. Real scans 78 with foreign material and foreign material artefacts are inputted into the trained MAR model 76, and the trained MAR model 76 generates predicted foreign material and foreign material artefact-free (MAR) scans 80 from those real scans 78. The predicted MAR scans 80 and the real foreign material-free scans 66 are inputted into discriminator network 64. (It will be noted that, in this embodiment, the real foreign material-free scans used in the MAR network training are the same foreign material-free scans as those used in the MAR model optimization but, in some other embodiments, the real foreign material-free scans used in the MAR network training are different from the foreign material-free scans used in the MAR model optimization.) Discriminator network 64 attempts to discriminate the predicted MAR scans 80 from the real foreign material and foreign material artefact-free scans 66. If differences between the predicted MAR scans 80 and real foreign material-free scans 66 are significant, discriminator network 64 is able to differentiate between these scans and, in response, the MAR network 62 fine-tunes itself to produce an improved trained MAR model 76. The MAR model 76 is repeatedly improved through this optimization procedure until MAR model 76 generates predicted MAR scans 80 that discriminator network 64 cannot be differentiated from the real foreign material and foreign material artefact-free scans 66.

MAR network 62 can be implemented in various designs. In one embodiment, MAR network 62 is implemented as a U-shape network. In another embodiment, MAR network 62 is implemented as a ResNet (residual neural network). The discriminator network 64 can also be implemented in various designs: in one embodiment, the discriminator network 64 is implemented as a GAN network. In another embodiment, the discriminator network 64 is implemented as an autoencoder network.

In this embodiment, MAR model 76 is trained from MAR network 62, then optimized by the discriminator network 64, such as by optimizing the MAR model 76 only after all the epochs of the MAR network training have been completed. In another embodiment, the MAR training and optimization happen concurrently with, for example, the MAR model optimized after each epoch of the MAR network has been completed.

In one embodiment, one model 46a for removing foreign material and foreign material artefacts is trained, such that the trained MAR model 76 removes both the foreign material and foreign material artefact from scans. In another embodiment, two models 47b, 47c are trained for removing foreign material artefacts and foreign material separately. For example, model 47b for foreign material artefact removal removes the foreign material artefacts only from a scan. The generated scan leaves the foreign material (e.g. metal) in its original place. A model 47c for foreign material removal could then be used to remove the foreign material from the scans. In one embodiment, the foreign material-removed region on the image is filled by a mask. In another embodiment, the foreign material-removed region on the image is filled by the predicted pixel/voxel values of human tissue.

In the embodiment illustrated in Figure 3A, deep learning architecture 60 has both an MAR network 62 and a discriminator network 64. In another embodiments, the deep learning architecture may have a MAR network 62 only. Hence, discriminator network 64 should be regarded as advantageous but optional.

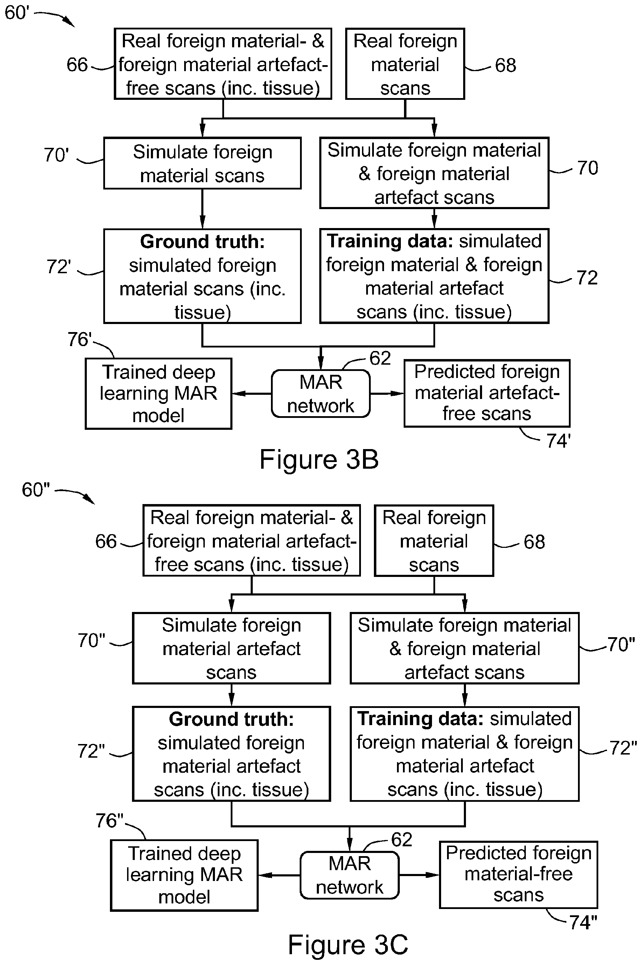

For example, Figure 3B depicts a deep learning architecture 60' (implemented by deep learning model trainer 30) that trains models 47b for foreign material artefact removal. Deep learning architecture 60' of Figure 3B includes one network MAR network 62, which outputs trained models 47b in a method that involves comparing predicted scans made by MAR network 62 with corresponding simulated scans, as follows.

Firstly, image simulator 28 uses real foreign material and foreign material artefact-free scans 66 and real foreign material scans 68, both to generate 70 simulated scans 72 with foreign material and foreign material artefacts and to generate 70' simulated scans 72' with foreign material. The simulated scans 72 with foreign material and foreign material artefacts may be regarded-in this example-as training data, while the simulated scans 72' with foreign material may be regarded as the ground truth.

The simulated scans 72 with foreign material and foreign material artefacts are annotated (using annotator 31), then inputted into MAR network 62. The output of MAR network 62 comprises predicted foreign material artefact-free scans 74'. MAR network 62 trains a deep learning MAR model 76' by minimizing the difference between the predicted scans 74' and the simulated scans 72' with foreign material.

In another example, Figure 3C depicts a deep learning architecture 60" (implemented by deep learning model trainer 30) that trains models 47c for foreign material removal. Deep learning architecture 60" of Figure 3C includes one network MAR network 62, which outputs trained models 47c by comparing predicted scans made by MAR network 62 with corresponding simulated scans, as follows.

Firstly, image simulator 28 uses real foreign material and foreign material artefact-free scans 66 and real foreign material scans 68, both to generate 70 simulated scans 72 with foreign material and foreign material artefacts and to generate 70" simulated scans 72" with foreign material artefacts. The simulated scans 72 with foreign material and foreign material artefacts may be regarded-in this example-as training data, while the simulated scans 72" with foreign material artefacts may be regarded as the ground truth.

The simulated scans 72 with foreign material and foreign material artefacts are annotated (using annotator 31), then inputted into MAR network 62. The output of MAR network 62 comprises predicted foreign material-free scans 74". MAR network 62 trains a deep learning MAR model 76" by minimizing the difference between the predicted scans 74" and the simulated scans 72" with foreign material artefacts.

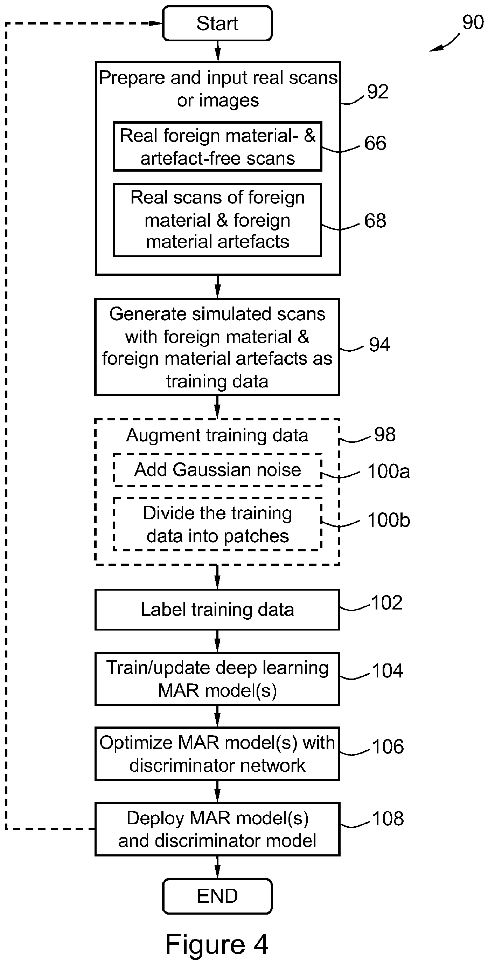

Figure 4 is a schematic flow diagram 90 of the training of a deep learning MAR model 76 for both foreign material and foreign material artefact removal. Firstly, at step 92, real data are prepared and inputted by system 10 (or inputted only by system 10, if already available). The real data include both real foreign material-free (and hence artefact-free) scans 66, in this example in the form of metal-free scans, and real scans 68 of foreign material and foreign material artefacts, in this example in the form real scans of metal and metal artefacts.

At step 94, on the basis of scans 66, 68, training data in the form of simulated scans are generated by simulating the foreign material and foreign material artefacts (in this example in the form of metal and metal artefacts) on or in the foreign material-free (in this example, metal-free) scans. That is, each simulated scan is generated by adding foreign material and foreign material artefacts on or to the images of a real foreign material-free scan. In another embodiment, the sinogram of a foreign material image and the sinogram of a real foreign material-free scan are combined; the combined sinogram is then converted to the simulated foreign material and foreign material artefact scans.

The method may optionally include a data augmentation step (step 98) to improve the training data 94. For example, this may involve adding Gaussian noise 100a to the training data to improve the robustness of the model training, and/or dividing the training data into patches 100b to increase the amount of training data.

At step 102, the training data scans are then labelled (typically manually) with labels or annotations that annotate, for example, the foreign material, the foreign material artefacts and tissues (such as bone) visible in the training data scans, that is, the real training data (cf. step 92) and the simulated training data (cf. step 94).

At step 104, the deep learning MAR model(s) 76 are trained or, if already trained, updated, by the MAR network using some of the training data. The training data includes real foreign material and foreign material artefact-free scans, real foreign material and foreign material artefact scans, and simulated foreign material and foreign material artefact scans. MAR network 62 trains/updates 104 the MAR model(s) 76 using predicted foreign material and foreign material artefact-free scans (generated from the simulated foreign material and foreign material artefact scans 72) and the real foreign material and foreign material artefact-free scans, and minimizes the difference therebetween.

At step 106, the deep learning MAR model(s) 76 are optimized with the discriminator network, using real metal and metal artefact-free scans and predicted metal and metal artefact-free scans (the latter generated from real metal and metal artefact scans). As mentioned above, in this embodiment the real metal and metal artefact-free scans used in the MAR network training are the same metal-free scans used in the MAR model optimization, but in other embodiments, the real metal and metal artefact-free scans used in the MAR network training may be different from the real metal-free scans used in the MAR model optimization.

Also as noted above, in this embodiment the deep learning MAR model is trained from the MAR network, then optimized by the discriminator network, such as by optimizing the MAR model after all the epochs of the MAR network training are complete. In another embodiment, the MAR training and optimization are conducted concurrently, such as by optimizing the MAR model after each epoch of the MAR network has been completed.

In one embodiment, one model of removing metal and metal artefact is trained. The trained model removes both the metal and metal artefact from scans. In another embodiment, two models are trained for removing metal and metal artefacts separately with, for example, the metal artefact removal model removing only metal artefacts from scans (the generated scan keeping the metal in its original place). The metal removal model can then be used to remove the metal from those scans.

At step 108, the trained MAR model(s) 76 and a discriminator model trained by the discriminator network are deployed, and stored in trained deep learning model store 46.

In use, a scan with metal and metal artefact is fed into the trained MAR model(s). The model processes the scan and outputs a scan with metal and metal artefact removed. The discriminator model is not used for metal and metal artefact removal. The discriminator model is stored for model optimization in further training.

When the MAR models are trained, additional training data might be collected to update the models. In one embodiment, only the MAR network is updated with the additional training data. In another embodiment, only the discriminator network is updated with the additional training data. In another embodiment, both the MAR network and the discriminator network are updated with the additional training data.

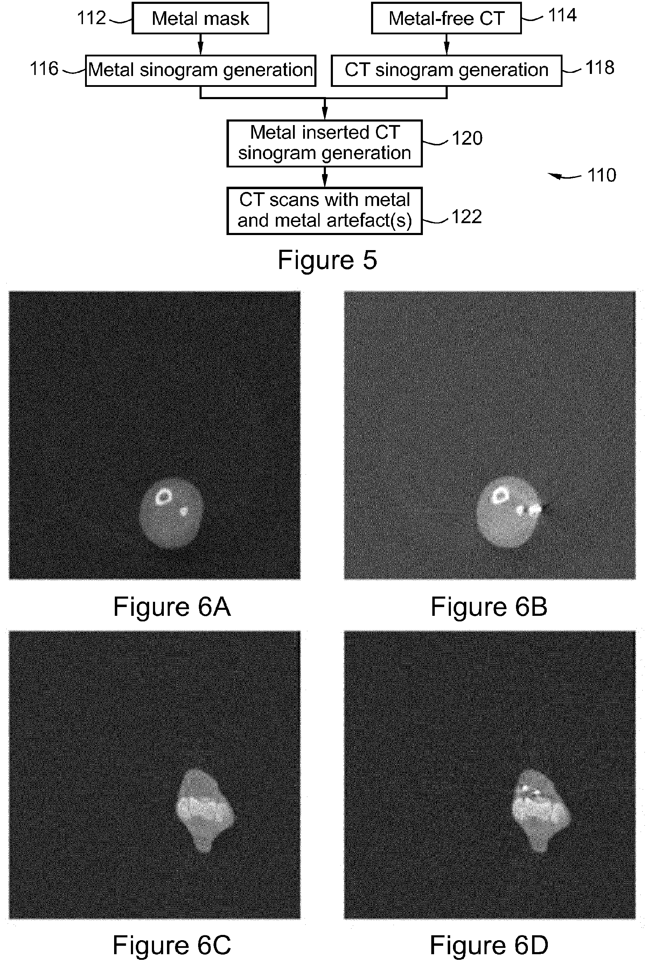

Figure 5 is a schematic flow diagram 110 of exemplary generation of a simulated metal and metal artefact scan. Referring to Figure 5, a simulated metal and metal artefact scan is generated through sinogram conversion. Through Radon transform, a metal mask image and a metal-free CT scan are converted to the metal sinogram and CT sinogram, respectively. The metal sinogram is overlapped on the CT sinogram to generate the sinogram of the metal-inserted CT. The metal-inserted CT sinogram is converted to an image using inverse Radon transform. The image is the CT image with the simulated metal and metal artefact.

In one embodiment, the metal mask image is generated by segmenting the metal region from real CT scans with metal. In another embodiment, the metal mask image is generated by simulation, such as by placing clusters of bright pixels/voxels are placed on a black or blank image.

In one embodiment, the metal mask image has one metal region. In another embodiment, the metal mask image has more than one metal region.
Figures 6A to 6D show examples of real metal-free CT and simulated metal and metal artefact CT scans. Figures 6A and 6C are real metal-free CT scans of a lower leg in cross-section, the latter being more distal (viz. closer to the ankle). Figures 6B and 6D are corresponding simulated CT scans including metal and metal artefacts. The simulated scans are generated according to the implementation described in Figure 5. In Figures 6B and 6D, the metal in the simulated scans appears as clusters of bright pixels/voxels, to the right of the fibula in Figure 6B and above the tibia in Figure 6D; the metal artefacts appear as beam hardening, scattering (which causes ray-like effects) and photon starvation (which causes dark spots).

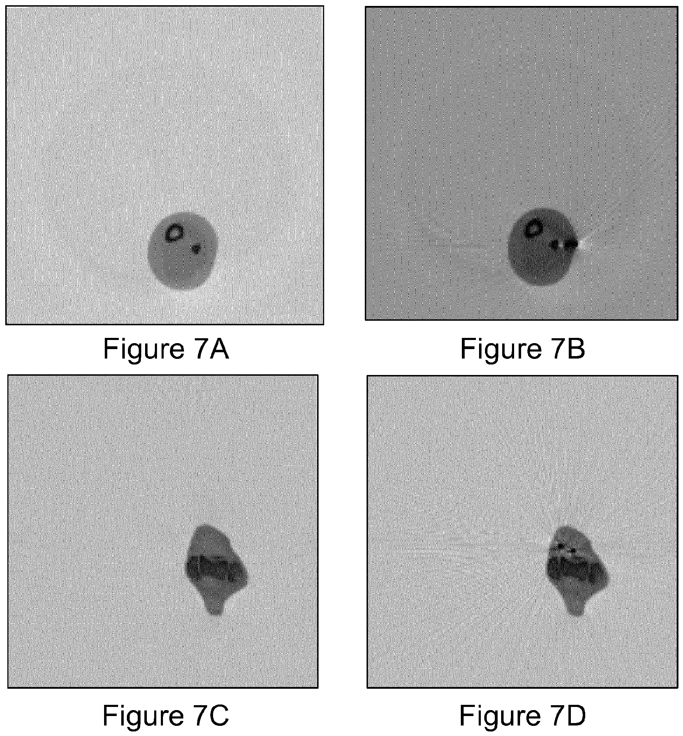

Figures 7A to 7D are inverted versions of the images of Figures 6A to 6D, respectively, and are provided to aid the interpretation of Figures 6B and 6D. In Figures 7B and 7D, the metal in the simulated scans appears as clusters of dark pixels/voxels, and the regions of photon starvation and bright areas.

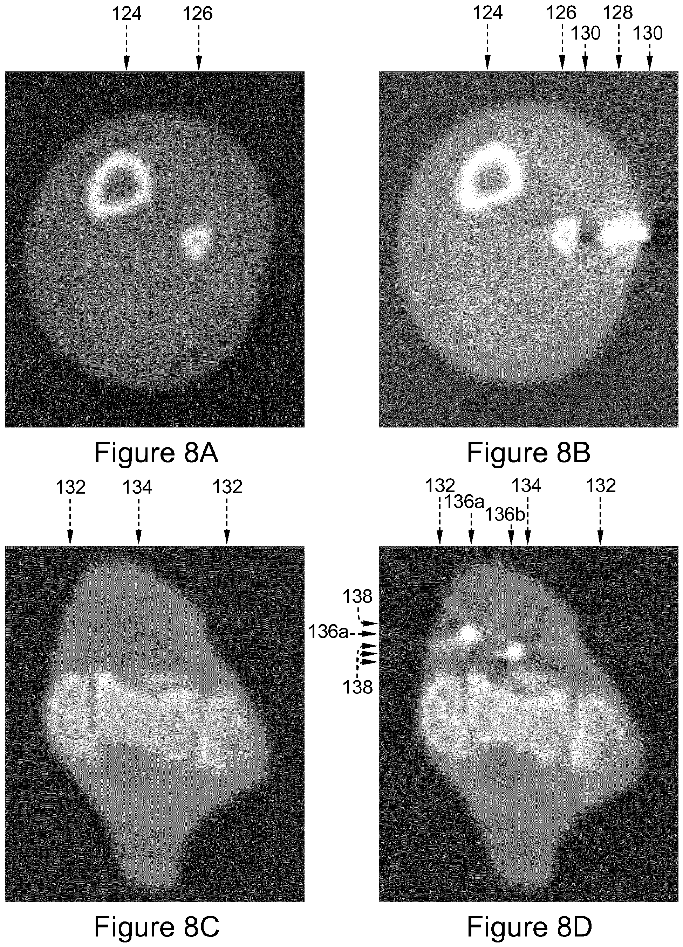

Figures 8A to 8D are enlarged details of the leg portions of the images of Figures 6A to 6D, respectively. In Figures 8A and 8B the tibia is indicated by 124 and the fibula by 126, while-in Figure 8B-metal indicated by 128 and photon starvation artefacts by 130. In Figures 8C and 8D the tibia is indicated by 132 and the talus by 134, while-in Figure 8B-metal is indicated by 136a and 136b and photon starvation artefacts by 138.

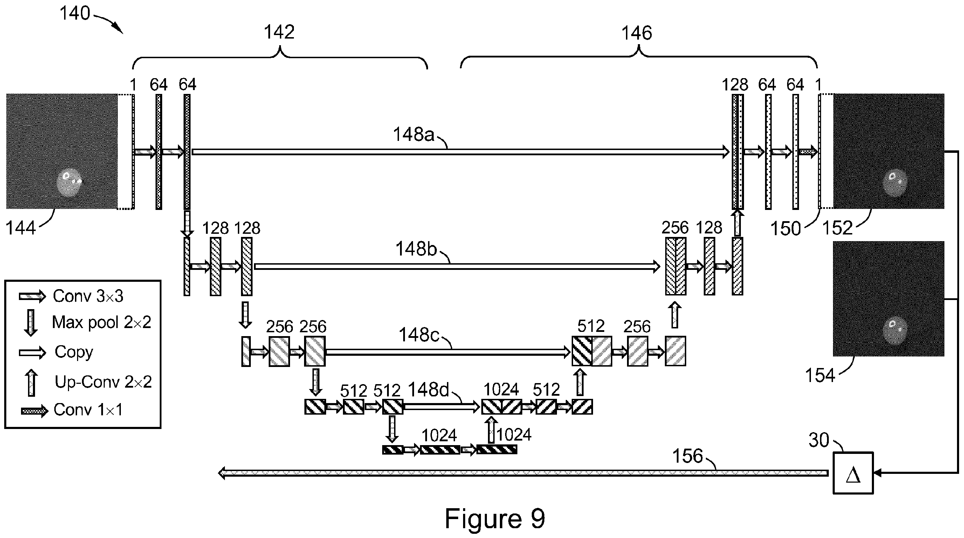

Figure 9 depicts an exemplary implementation 140 (with exemplary inputs and outputs) of the metal and metal artefact removal network 62 of Figure 3A. Referring to Figure 9, MAR network 140 includes a contracting part 142 (which receives scan images with metal and metal artefacts 144 as input) and an expansive part 146. Contracting part 124 includes four layers, each of which contains two 3×3 convolution layers and one 2×2 max-pooling layer. Expansive part 146 also includes four layers, with each layer containing a 2×2 up-convolution layer and two 3×3 convolution layers. The two parts 142, 146 are connected via respective skip-connections 148a, 148b, 148c, 148d in the various corresponding layers of the network 140. The feature maps in each layer of contracting part 142 are concatenated with the corresponding layer in the expansive part 146 via the respective skip-connection 148a, 148b, 148c, 148d. The output of the last layer of contracting part 142 is connected to the beginning of expansive part 146 via two 3×3 convolution layers. At the last layer of expansive part 146, a 1×1 convolution layer 150 is used to generate predicted images 152 with the metal and metal artefact removed. The input data dimension in this example is 1×480×480, which is the same as the output. The feature map dimensions and numbers are also indicated in the figure. The feature map number is doubled, and the feature map size is reduced by half between each layer of contracting part 142, while in expansive part 146 the feature map number is reduced by half, and the feature map size is doubled between each layer.

During the training of a MAR model, the inputs 144 are simulated foreign material and foreign material artefact scans, and the outputs are predicted MAR scans 152. The ground truth data 154 are real foreign material-free scans, from which the simulated foreign material and foreign material artefact scans are generated. MAR model trainer 32 subjects the ground truth data 154 and the predicted MAR scans 152 to a mean square loss calculation. The resulting mean square loss is used to update the model parameters by loss backpropagation 156.

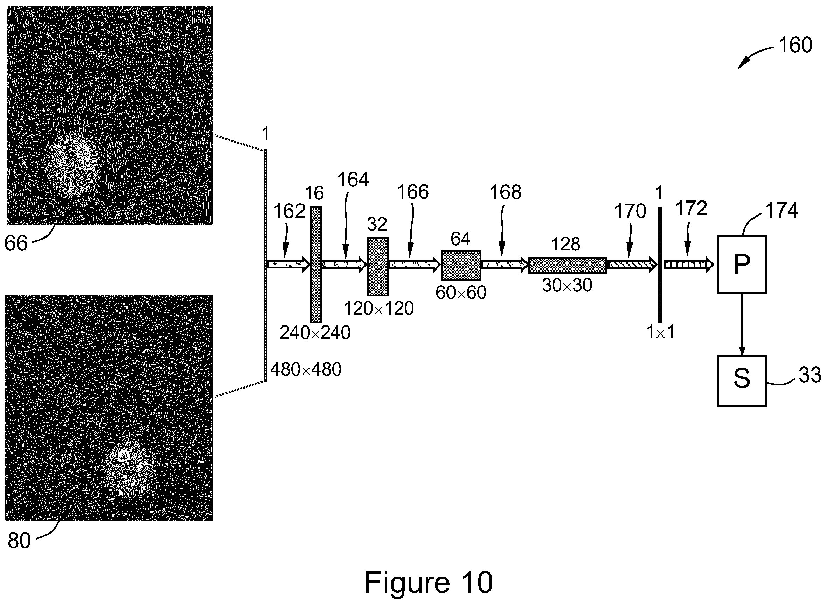

Figure 10 depicts an exemplary implementation 160 (with exemplary inputs and outputs) of discriminator network 64 of Figure 3A. Referring to Figure 10 (and as discussed above), discriminator network receives one or more real foreign material and foreign material artefact-free scans 66 and one or more predicted MAR scans 80, and uses four convolution modules 162, 164, 166, 168 to extract the image feature from the respective input image 66, 80, followed by a flattening layer 170 and a fully connected layer 172. Each convolution module 162, 164, 166, 168 includes a 3×3 convolution layer, a ReLU layer, and a BatchNorm layer.

The output of fully connected layer 172 of discriminator network 160 is probability data 174 that indicates whether the input data was a real scan 66 or a predicted scan 80. In addition, discriminator model trainer 33 determines the performance of the discriminator network by determining and outputting cross-entropy loss, where the greater the cross-entropy loss, the greater the likelihood that the discriminator has made an incorrect identification. This can then be used to train the discriminator network (or, more specifically, the discriminator model trained by the discriminator network).

Thus, the MAR models are configured to generate MAR predictions as similar as possible to the real foreign material and foreign material artefact-free scans, and the discriminator aims to distinguish real foreign material and foreign material artefact-free scans from predicted MAR scans. It will be appreciated that, as explained above, the MAR network of Figures 9 and 10 may be implemented in a variety of alternative designs, such as a U-shape network or as a ResNet.

As also mentioned above, a foreign material and foreign material artefact removal model adapted to remove both foreign material (e.g. metal) and artefacts of the foreign material can comprise two models, a first model 47b trained to remove artefacts of the foreign material(s) and a second model 47c trained to separately remove the foreign material(s). Each of those separate models can be trained by using the deep learning architecture of Figure 3A but varying its inputs as required.

Indeed, in some applications it may be desired to train and employ an foreign material artefact removal model, adapted to remove only the foreign material artefacts (but not the foreign material) from a scan, whether for use alone or in conjunction with a foreign material removal model. If training data is used in the form of images/scans with only artefacts removed, the model, once trained, will be adapted to remove artefacts only-despite the presence in the original images or scans of both the foreign material and artefacts of the foreign material.

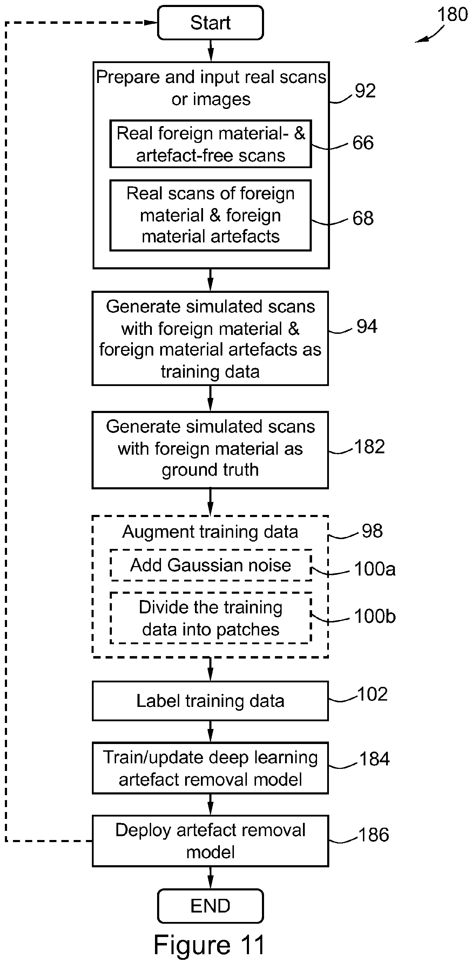

Figure 11 is a schematic flow diagram 180, comparable to that of Figure 4, of the training of an alternative deep learning model 76 in the form of a deep learning artefact removal model 47b for removing artefacts of a foreign material such as metal. Flow diagram 180 is similar to flow diagram 90 of Figure 4, and like reference numerals have been used to identify like features.

Firstly, at step 92, real data are prepared and inputted by system 10 (or inputted only by system 10, if already available). The real data include both real foreign material-free (and hence artefact-free) scans 66, in this example in the form of metal-free scans, and real scans 68 with foreign material and foreign material artefacts, in this example in the form real scans of metal and metal artefacts.

At step 94, on the basis of scans 66, 68, training data is generated in the form of simulated foreign material and foreign material artefact scans. At step 182, on the basis of scans 66, 68, ground truth data is generated in the form of simulated foreign material scans.

The method may optionally include a data augmentation step (step 98) to improve the training data 94. For example, this may involve adding Gaussian noise 100a to the training data to improve the robustness of the model training, and/or dividing the training data into patches 100b to increase the amount of training data.

At step 102, the training data scans are then labelled (typically manually) with labels or annotations that annotate, for example, the foreign material, the foreign material artefacts and tissues (such as bone) visible in the training data scans, that is, the first set of training data (cf. step 92) and the simulated training data (cf. step 184).

At step 184, the deep learning artefact removal model(s) 76 are trained or, if already trained, updated, by the MAR network using some or all of the training data and the ground truth data.

At step 186, the trained artefact removal model(s) 76 are deployed, and stored in trained deep learning model store 46.

### Example

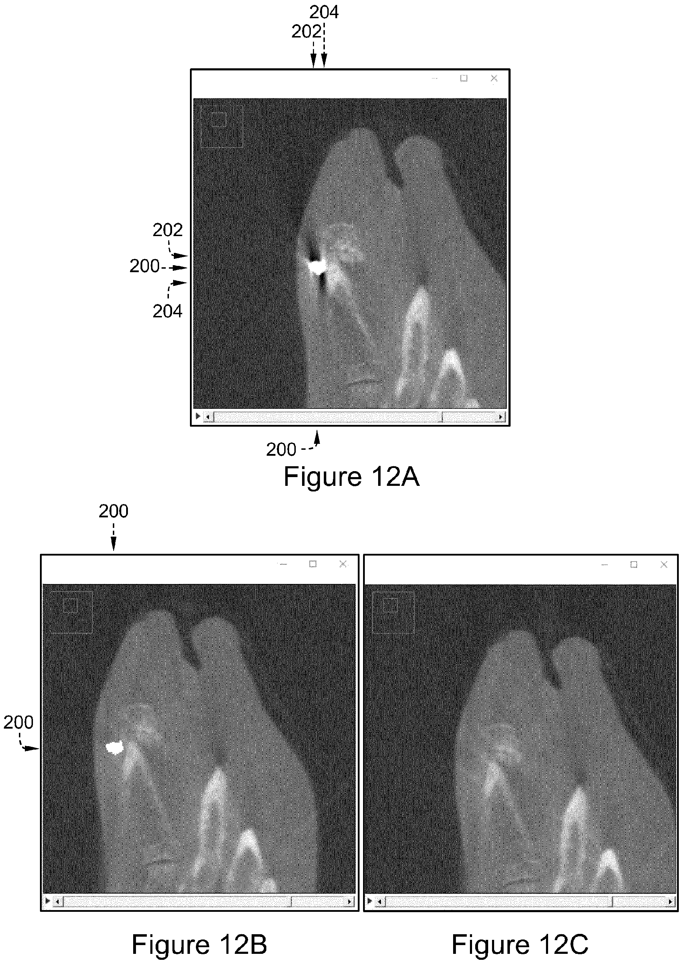

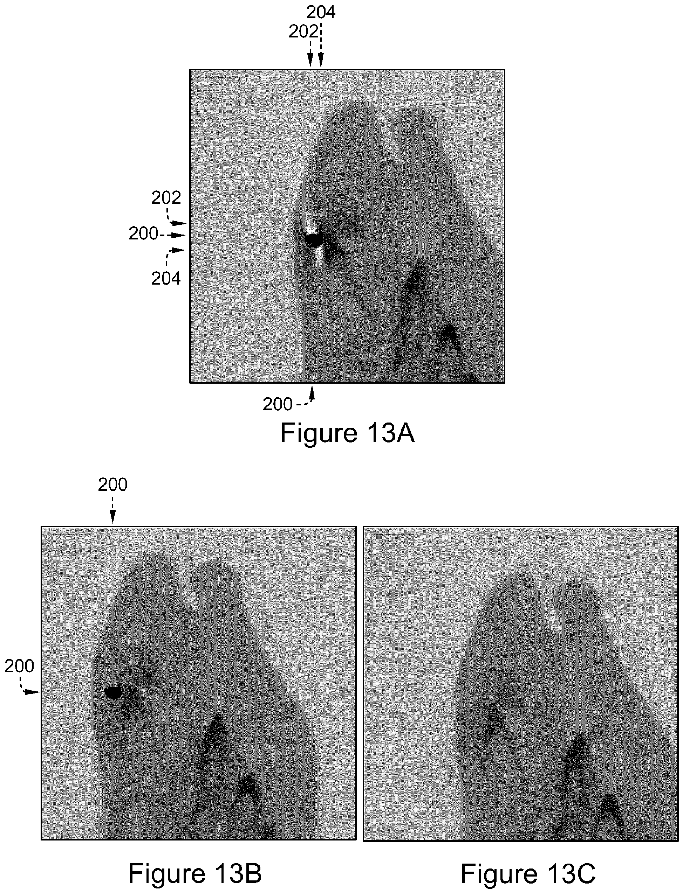

Figures 12A, 12B and 12C are, respectively, a CT image of a foot in raw form, the same image with metal artefacts (due to the presence of steel) removed using a metal artefact removal model according to an embodiment of the invention, and the same image with both metal and metal artefacts removed using a MAR model according to an embodiment of the invention. (Figures 13A, 13B and 13C are inverted versions of the images of Figures 12A, 12B and 12C, respectively, presented for clarity.) As can be seen in Figure 12A, the original image includes a cluster 200 of bright pixels/voxels, due to the presence of metal. Metal artefacts include a first dark area 202 adjacent to and above the metal and a second dark area 204 adjacent to and below the metal (due to photon starvation), and surrounding dark rays.

As can be seen in Figure 12B, the metal artefacts (including first and second dark areas 202, 204) have been greatly reduced and the original image in the affected areas largely restored. As can be seen in Figure 12C, the metal artefacts and the bright cluster 200 due to metal have been greatly reduced and the affected areas largely restored.

It will be understood by persons skilled in the art of the invention that many modifications may be made without departing from the scope of the invention. In particular it will be apparent that certain features of embodiments of the invention can be employed and combined to form further embodiments.

It is to be understood that, if any prior art is referred to herein, such reference does not constitute an admission that the prior art forms a part of the common general knowledge in the art in any country.

In the claims that follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

### References:

[1] Park, H. S., Hwang, D., & Seo, J. K. (2015). Metal artefact reduction for polychromatic X-ray CT based on a beam-hardening corrector. IEEE transactions on medical imaging, 35(2), 480-487.
[2] Zhang, Y., Mou, X., & Tang, S. (2010, October). Beam hardening correction for fan-beam CT imaging with multiple materials. In IEEE Nuclear Science Symposium & Medical Imaging Conference (pp. 3566-3570).
[3] Mehranian, A., Ay, M. R., Rahmim, A., & Zaidi, H. (2013). X-ray CT metal artefact reduction using wavelet domain L0 sparse regularization. IEEE transactions on medical imaging, 32(9), 1707-1722.
[4] Zhang, Y., Pu, Y. F., Hu, J. R., Liu, Y., Chen, Q. L., & Zhou, J. L. (2011). Efficient CT metal artefact reduction based on fractional-order curvature diffusion. Computational and mathematical methods in medicine, 2011.
[5] Wang, G., Snyder, D. L., O'Sullivan, J. A., & Vannier, M. W. (1996). Iterative deblurring for CT metal artefact reduction. IEEE transactions on medical imaging, 15(5), 657-664.
[6] Wang, G., Vannier, M. W., & Cheng, P. C. (1999). Iterative X-ray cone-beam tomography for metal artefact reduction and local region reconstruction. Microscopy and microanalysis, 5(1), 58-65.
[7] Zhang, Y., & Yu, H. (2018). Convolutional neural network based metal artefact reduction in x-ray computed tomography. IEEE transactions on medical imaging, 37(6), 1370-1381.
[8] Liao, H., Lin, W. A., Zhou, S. K., & Luo, J. (2019). ADN: artefact disentanglement network for unsupervised metal artefact reduction. IEEE Transactions on Medical Imaging, 39(3), 634-643.

## Claims

1. A method for training a machine learning model for reducing or removing at least one foreign material or artefacts due to the foreign material from an image, the method comprising:
generating one or more first simulated images from one or more real or simulated images of the foreign material, and from one or more real images of one or more subjects that are free of the foreign material and of artefacts due to the foreign material, such that the generated simulated images include the foreign material and artefacts due to the foreign material;
generating one or more predicted images employing at least the first simulated images with a machine learning network that implements a machine learning model; and
training or updating the machine learning model with the machine learning network by reducing or minimizing a difference between the one or more predicted images and ground truth data comprising one or more real or simulated images.

2. The method of claim 1, wherein the one or more real or simulated images of the foreign material include artefacts due to the foreign material.

3. The method of either claim 1 or 2, wherein the one or more predicted images are free of both the foreign material and artefacts due to the foreign material, and the ground truth data comprises one or more real images of one or more subjects that are free of the foreign material and of artefacts due to the foreign material, and the machine learning model is configured to reduce or remove a foreign material and artefacts due to a foreign material from an image.

4. The method of either claim 1 or 2, further comprising:
generating one or more second simulated images from the one or more real or simulated images of the foreign material, and from the one or more real images of the one or more subjects that are free of the foreign material and of artefacts due to the foreign material, such that the generated second simulated images include the foreign material; wherein the one or more predicted images are free of artefacts due to the foreign material, the ground truth data comprises the second simulated images, and the machine learning model is configured to reduce or remove artefacts due to the foreign material from an image; or
generating one or more second simulated images from the one or more real or simulated images of the foreign material, and from the one or more real images of the one or more subjects that are free of the foreign material and of artefacts due to the foreign material, such that the generated second simulated images include artefacts due to the foreign material; wherein the one or more predicted images are free of the foreign material, the ground truth data comprises the second simulated images, and the machine learning model is configured to reduce or remove the foreign material from an image.

5. The method as claimed in any one of the preceding claims, wherein the foreign material is titanium alloy, cobalt-chromium alloy, steel, stainless steel, dental amalgam, silver or other metal; or the foreign material is a ceramic, glass, polymeric, a composite, a glass-ceramic, or a biomaterial.

6. The method as claimed in any one of the preceding claims, wherein the machine learning model is configured to reduce or remove a plurality of foreign materials and/or artefacts due to the foreign materials from an image.

7. The method as claimed in any one of the preceding claims, further comprising annotating or labelling the one or more first simulated images.

8. A system for training a machine learning model for reducing or removing at least one foreign material or artefacts due to the foreign material from an image, the system comprising:
an image simulator configured to generate one or more first simulated images from one or more real or simulated images of the foreign material, and from one or more real images of one or more subjects that are free of the foreign material and of artefacts due to the foreign material, such that the generated simulated images include the foreign material and artefacts due to the foreign material;
a machine learning network configured to generate one or more predicted images employing at least the first simulated images, the machine learning network implementing a machine learning model;
wherein the machine learning network is configured to reduce or minimize a difference between the one or more predicted images and ground truth data comprising one or more real or simulated images.

9. The system of claim 8, wherein the one or more real or simulated images of the foreign material include artefacts due to the foreign material.

10. The system of either claim 8 or 9, wherein the one or more predicted images are free of both the foreign material and artefacts due to the foreign material, and the ground truth data comprises one or more real images of one or more subjects that are free of the foreign material and of artefacts due to the foreign material, such that the machine learning model is configured to reduce or remove a foreign material and artefacts due to a foreign material from an image.

11. The system of either claim 8 or 9, wherein the image simulator is further configured to:
generate one or more second simulated images from the one or more real or simulated images of the foreign material, and from the one or more real images of the one or more subjects that are free of the foreign material and of artefacts due to the foreign material, such that the generated second simulated images include the foreign material; wherein the one or more predicted images are free of artefacts due to the foreign material, and the ground truth data comprises the second simulated images, such that the machine learning model is configured to reduce or remove artefacts due to the foreign material from an image; or.
generate one or more second simulated images from the one or more real or simulated images of the foreign material, and from the one or more real images of the one or more subjects that are free of the foreign material and of artefacts due to the foreign material, such that the generated second simulated images include artefacts due to the foreign material; wherein the one or more predicted images are free of the foreign material, and the ground truth data comprises the second simulated images, such that the machine learning model is configured to reduce or remove the foreign material from an image.

12. The system as claimed in any one of claims 8 to 11, wherein the foreign material is titanium alloy, cobalt-chromium alloy, steel, stainless steel, dental amalgam, silver or other metal; or the foreign material is a ceramic, glass, polymeric, a composite, a glass-ceramic, or a biomaterial.

13. The system as claimed in any one of claims 8 to 12, wherein the system is configured to train the machine learning model to reduce or remove a plurality of foreign materials and/or artefacts due to the foreign materials from an image.

14. The system as claimed in any one of claims 8 to 13, further comprising an annotator configured or operable to receive annotations or labels for features of the one or more first simulated images.

15. A method for reducing or removing at least one foreign material or artefacts due to the foreign material from an image, the method comprising:
reducing or removing from an image of a subject at least one foreign material or artefact due to the foreign material, or both the at least one foreign material and the artefact due to the foreign material, using a machine learning model trained according to the method of any one of claims 1 to 7.

16. A system for reducing or removing at least one foreign material or artefacts due to the foreign material from an image, the system being configured to reduce or remove from an image of a subject at least one foreign material or artefact due to the foreign material, or both the at least one foreign material and the artefact due to the foreign material, using a machine learning model trained according to the method of any one of claims 1 to 7.

17. A computer program comprising program code configured, when executed by one of more computing devices, to implement the method of any one of claims 1 to 7.

18. A computer-readable medium, comprising the computer program of claim 17.
